(19) Europäisches Patentamt
European Patent Office
Office européen des brevets

(11) EP 2 068 958 B1

(12) **EUROPEAN PATENT SPECIFICATION**

(45) Date of publication and mention of the grant of the patent:
**19.10.2016 Bulletin 2016/42**

(21) Application number: **07838950.9**

(22) Date of filing: **27.09.2007**

(51) Int Cl.:
***A61L 31/10*** *(2006.01)* ***A61L 31/06*** *(2006.01)*
***A61L 31/16*** *(2006.01)* ***A61L 31/02*** *(2006.01)*

(86) International application number:
**PCT/US2007/020872**

(87) International publication number:
**WO 2008/042227 (10.04.2008 Gazette 2008/15)**

(54) **MEDICAL DEVICE INCLUDING AN ANESTHETIC AND METHOD OF PREPARATION THEREOF**

MEDIZINISCHE VORRICHTUNG MIT EINEM ANÄSTHETIKUM UND VERFAHREN ZU IHRER HERSTELLUNG

DISPOSITIF MÉDICAL COMPRENANT UN ANESTHÉSIQUE ET SON PROCÉDÉ DE FABRICATION

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **28.09.2006 US 847841 P**

(43) Date of publication of application:
**17.06.2009 Bulletin 2009/25**

(73) Proprietor: **Cook Medical Technologies LLC**
**Bloomington, IN 47404 (US)**

(72) Inventors:
- **ISCH, Andrew, P.**
**Lafayette, IN 47909 (US)**
- **RUANE, Patrick, H.**
**Hayward, CA 94544 (US)**
- **PEREZ-SEGARRA, Waleska**
**West Lafayette, IN 47906 (US)**

(74) Representative: **Garratt, Peter Douglas et al**
**Mathys & Squire LLP**
**The Shard**
**32 London Bridge Street**
**London SE1 9SG (GB)**

(56) References cited:
**US-A1- 2004 006 381** **US-B1- 6 368 658**
**US-B1- 6 395 326**

EP 2 068 958 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD

The technical field of the patent is that of medical devices containing an anesthetic and processes for incorporating an anesthetic into such devices.

### BACKGROUND

It has become common to treat a variety of medical conditions by introducing an implantable medical device partly or completely into the esophagus, trachea, colon, biliary tract, urinary tract, or other location within a human or veterinary patient. For certain applications, the medical device includes an anesthetic and is adapted to expose tissue within the body to the anesthetic over a desired time interval, such as by releasing the anesthetic. Desirably, the anesthetic is released within the body at a reproducible and predictable fashion so as to optimize the benefit of the anesthetic to the patient over the desired period of time.

Providing medical devices adapted to release an anesthetic at a desired rate over a period of time is one challenge in designing implantable medical devices. For example, a medical device may release an anesthetic at a greater rate than desired upon implantation, and subsequently release the anesthetic at a slower rate than desired at some time after implantation. What is needed is a medical device that provides for release of one or more anesthetics over a period of time, desirably at an optimal elution rate from the device.

Many drugs exist in a non-ionic form that is either insoluble or sparingly soluble in an aqueous environment. Because of this limited solubility, such drugs are often administrated systemically in a more soluble ionic form, such as a hydrochloride or sodium salt and so on. For example, U.S. Pat. No. 4,795,644 teaches that if a drug is not sufficiently soluble at a desired concentration, derivatives of the drug, such as its hydrochloride or sodium salt may be used instead in order to prepare a physiologically active coating, in combination with a resin and a water-leachable wall surrounding the agent and the resin.

Other processes, such as those for self-assembling monolayers (SAM), provide an ionic surface coating or function layer on the surface of a medical device. Exemplary of this approach are U.S. Patent Nos. 5,759,708, and 5,958,430. The medical device is then prepared by repeated sprayings or coatings onto the surface. One disadvantage of these processes is that the resulting coating may appear to be uneven and crystalline, as though coated with crystalline salt. Other disadvantages are that the coating thickness be somewhat variable and the coating itself may be physically weak. In some instances, the coating itself lacks integrity and may be difficult to retain on the surface of the medical device.

Another process is disclosed in U.S. Publication No. 2005/0025791 A1, published February 3, 2005. This publication teaches screening a plurality of pharmaceutical solutions, typically metal salts of an active pharmaceutical ingredient, such as alkali metal salts or alkaline earth salts, and selecting the ones with the desired performance. This process continues to rely on ionic forms of the pharmaceutical. Because of this, the resulting coating may not be as adherent as desired and therefore may be relatively unstable in an aqueous environment.

### BRIEF SUMMARY

One aspect provides a non-metallic medical device having an anesthetic in contact with the non-metallic substrate. The anesthetic has a proton binding site with a non-ionic form and an ionic form, the anesthetic being less soluble in water when the proton binding site is in the non-ionic form than when the proton binding site is in the ionic form. In one embodiment, at least 5% w/w of the anesthetic is present with the proton binding site in the non-ionic form and the remainder of the anesthetic is present in the ionic form. In another embodiment, at least 95% w/w of the anesthetic is present with the proton binding site in the non-ionic form and the remainder of the anesthetic is present in the ionic form.

In one embodiment, the proton binding site has a protonated ionic form and an unprotonated non-ionic form. In another embodiment, the proton binding site includes a nitrogen atom. In yet another embodiment, the proton binding site is a tertiary amine in the non-ionic form and a cationic quaternary amine in the ionic form. In another embodiment, the anesthetic comprises a chemical structure of the formula:

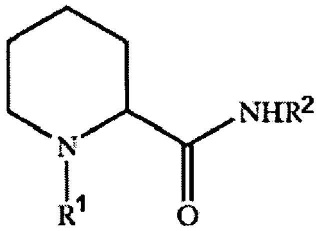

where $R^1$ is alkyl and $R^2$ is an optionally alkyl substituted phenyl.

In one embodiment, the anesthetic is bupivacaine, chloroprocaine, cocaine, lidocaine, mepivacaine, pramoxine or ropivacaine.

In another embodiment, the non-metallic substrate includes carbon, carbon fiber, cellulose acetate, cellulose nitrate, polyethylene teraphthalate, silicone, polyurethane, polyamide, polyester, polyorthoester, polyanhydride, polyether sulfone, polycarbonate, polypropylene, high molecular weight polyethylene, polytetrafluoroethylene, a biocompatible polymeric material, polylactic acid, polyglycolic acid, polyanhydride, polycaprolactone, polyhydroxybutyrate valerate, a protein, an extracellular matrix component, collagen, fibrin or mixtures or copolymers thereof. In a preferred embodiment the non-metallic substrate includes a polyurethane.

Another aspect provides a method for incorporating an anesthetic into a medical device. The method includes (a) dissolving an anesthetic in a solvent to form a solution, the anesthetic having a proton binding site for an acidic proton having a first pKa and having an ionic form and a non-ionic form in aqueous solution, the anesthetic being less soluble in water when the proton binding site is in the non-ionic form than when the proton binding site is in the ionic form, (b) maintaining the pH of the solution above the pKa of the acidic proton such that the solution contains the anesthetic with the proton binding site in the non-ionic form; and (c) contacting the medical device with the solution in a manner effective to incorporate the anesthetic with the proton binding site in the non-ionic form into the medical device.

The method may also include (a) dissolving the anesthetic with the proton binding site in the ionic form in a first solvent to form a pre-coating solution having a first pH; (b) lowering the pH of the pre-coating solution to convert at least a portion of the anesthetic proton binding site from the ionic form to the non-ionic form; (c) isolating at least a portion of the anesthetic having the proton binding site in the non-ionic form from the pre-coating solution; and (d) dissolving the isolated portion of the anesthetic in the solvent to form the solution.

BRIEF DESCRIPTION OF THE DRAWINGS

**Figures 1-2A** depict medical devices useful in coated embodiments;
**Figure 3** is a graph depicting elution profiles of a coated stent embodiment. The graph shows the elution of bupivacaine in buffers having pH values of 4.5 (•), 7.0 (■) and 9.5 (▲) over a 12-day period;
**Figures 4-5** are illustrations depicting coatings of prior art pharmacologically active substances onto stents;
**Figures 6-7** are illustrations depicting stents having a coating of a non-ionic form of bupivacaine; and
**Figure 8** is a graph showing the elution of bupivacaine from coated polyurethane drainage stents.

DETAILED DESCRIPTION

Definitions

Unless otherwise defined, all technical and scientific terms used herein have the same meaning as commonly understood by one of ordinary skill in the art to which this invention pertains. In case of conflict, the present document, including definitions, will control. Preferred methods and materials are described below, although methods and materials similar or equivalent to those described herein can be used in the practice or testing of the present invention.

As used herein the terms "comprise(s)," "include(s)," "having," "has," "can," "contain(s)," and variants thereof, are intended to be open-ended transitional phrases, terms, or words that do not preclude the possibility of additional acts or structures. The present invention also contemplates other embodiments "comprising," "consisting of" and "consisting essentially of," the embodiments or elements presented herein, whether explicitly set forth or not.

The terms "about" or "substantially" used with reference to a quantity includes variations in the recited quantity that are equivalent to the quantity recited, such as an amount that is insubstantially different from a recited quantity for an intended purpose or function.

As used herein, the term "implantable" refers to an ability of a medical device to be positioned, partially or

wholly, at a location within a body of a human or veterinary patient for any suitable period of time, such as within a body vessel. Furthermore, the terms "implantation" and "implanted" refer to the positioning of a medical device, partially or wholly, at a location within a body, such as within a body vessel. Implantable medical devices can be configured for transient placement within a body vessel during a medical intervention (e.g., minutes to hours), or to remain in a body vessel for a prolonged period of time after an implantation procedure (e.g., weeks or months or years). Implantable medical devices can include devices configured for bioabsorption within a body during a prolonged period of time.

The term "biocompatible" refers to a material that is substantially non-toxic in the in vivo environment of its intended use, and that is not substantially rejected by the patient's physiological system (i.e., is non-antigenic). This can be gauged by the ability of a material to pass the biocompatibility tests set forth in International Standards Organization (ISO) Standard No. 10993 and/or the U.S. Pharmacopeia (USP) 23 and/or the U.S. Food and Drug Administration (FDA) blue book memorandum No. G95-1, entitled "Use of International Standard ISO-10993, Biological Evaluation of Medical Devices Part-1: Evaluation and Testing." Typically, these tests measure a material's toxicity, infectivity, pyrogenicity, irritation potential, reactivity, hemolytic activity, carcinogenicity and/or immunogenicity. A biocompatible structure or material, when introduced into a majority of patients, will not cause an undesirably adverse, long-lived or escalating biological reaction or response, and is distinguished from a mild, transient inflammation which typically accompanies surgery or implantation of foreign objects into a living organism.

As used herein, the phrase "controlled release" refers to the release of an anesthetic at a predetermined rate. A controlled release may be characterized by a drug elution profile, which shows the measured rate that the material is removed from a material-coated device in a given solvent environment as a function of time. A controlled release does not preclude an initial burst release associated with the deployment of the medical device. In some embodiments of the invention an initial burst, followed by a more gradual subsequent release, may be desirable. The release may be a gradient release in which the concentration of the anesthetic released varies over time or a steady state release in which the anesthetic is released in equal amounts over a certain period of time (with or without an initial burst release).

Implantable Medical Device Incorporating an Anesthetic

One aspect provides an implantable medical device ("medical device") allowing for the controlled release of an anesthetic into the adjacent or surrounding tissue upon implantation in a patient. In one embodiment, the anesthetic is a compound having a proton binding site having an ionic form and non-ionic form existing in equilibrium in an aqueous environment. References to "the proton binding site in the non-ionic form" and the "proton binding site in the non-ionic form" refer to one or more proton binding sites on the anesthetic that provide different water solubilities to the anesthetic in the ionic form and the non-ionic form.

In one embodiment, the anesthetic having the proton binding site in the ionic form is the more soluble form of the anesthetic. Preferably, the medical device is manufactured having at least a portion of the anesthetic present in the non-ionic form. In one embodiment, at least 5% wt/wt of the anesthetic in present in the non-ionic form. In other embodiments, at least 10%, 20%, 30%, 40%, 50%, 60%, 70%, 80%, 90%, 95%, 99% or 100% wt/wt of the anesthetic is present in the non-ionic form.

Preferably, the anesthetic is a compound having a greater water solubility when the proton binding site is in a protonated ionic form and a lower water solubility when the proton binding site is in a deprotonated non-ionic form. For example, the anesthetic may be a piperidine carboxanilide compound, such as a compounds comprising the chemical structure of formula (1) in an unprotonated and non-ionic form:

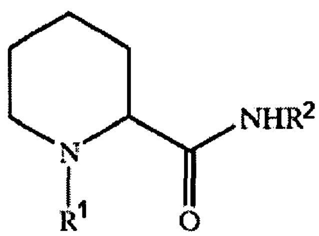

**(1)**

where $R^1$ is an alkyl group and $R^2$ is an optionally alkyl substituted aryl group. In formula (1), the proton binding site is preferably the tertiary amine bound to moiety $R^1$.

Medical devices including an anesthetic with such a proton binding site may offer several advantages. For example, incorporating required portions of the ionic form and the non-ionic form of the anesthetic allows for amounts of the anesthetic to be released for longer periods of time as compared to the release from previous devices.

As is discussed further below, many anesthetics are routinely administered systemically in an ionic form, in some cases because the non-ionic form is either insoluble or sparingly soluble in an aqueous environment. However, implanting a medical device having the anesthetic present mainly in the ionic form often results in the rapid release of the anesthetic from the device upon implantation.

Another undesirable aspect of using the ionic form of the anesthetic is that this form may be more hydrophilic than lipophilic. The non-ionic form may be more useful because it may be more easily absorbed by the tissue of a patient. In contrast, the more hydrophilic, ionic form may more easily dissolve in body fluids during its placement in the patient. In many cases, longer lasting, more uniform delivery of an anesthetic is achieved if an implantable medical device contains more of the non-ionic form than the ionic form.

For example, implantation of a medical device coated with an ionic form of the anesthetic drug bupivacaine (1-butyl-N-(2,6-dimethylphenyl)-2-piperidinecarboxamide hydrochloride) (i.e. Bupivacaine·HCl) may result in the majority of the drug being released into body fluids before the device reaches the desired implantation site.

When such an anesthetic is present with the proton binding site in the non-ionic form, release of the anesthetic from the medical device in an aqueous environment occurs over a significantly longer time period than when the anesthetic is present with the proton binding site in the ionic form. For example, **Figure 4,** which is discussed in Example 1, shows a release profile of a non-ionic form of the bupivacaine from a ureteral stent when the stent is placed in solutions having various pH values. Release occurs over a period of about 12 days. In comparison, when the medical device contains the ionic form, Bupivacaine.HCl, release occurs under the same conditions within a matter of minutes. Surprisingly, irrespective of the pH of the eluting solution, release of the non-ionic form occurs at approximately the same rate. Thus, use of the non-ionic form of an anesthetic for local delivery of the anesthetic offers the potential of a consistent rate of elution of the anesthetic over a range of pH values in the local environment of the medical device.

In one embodiment, medical devices incorporating an anesthetic with a proton binding site in the non-ionic form allow drug doses to be minimized to those necessary for treatment. Administering lesser quantities of anesthetics will tend to minimize any undesirable side effects of the anesthetics.

In another embodiment, medical devices having an anesthetic coated with a proton binding site in the non-ionic form are more durable than devices having ionic coatings. It has been found that a coating containing with the non-ionic form of an anesthetic can be far more adherent than a coating containing substantially the ionic form of the anesthetic. In some cases, the ionic form exists as a crystalline structure, resulting in a rough coating having poor adhesion properties, whereas the non-ionic form exists as a fine powder that forms a smoother coating having superior adhesion to the surface of the medical device.

In yet another embodiment, the present invention provides medical devices having a surface adapted for placement adjacent to or in contact with a lipophilic surface within a body (for example, a cellular surface having lipophilic cell membranes.) Contacting such a surface with a surface of a medical device containing a non-ionic form of an anesthetic may, in some cases, enhance absorption of the anesthetic into the cell by allowing the non-ionic form, which is often more lipophilic than the ionic form, to more easily cross the cell membrane and enter the cell before conversion to the active ionic form.

For example, if a surface of a medical device containing the non-ionic form of bupivacaine is positioned against a cellular surface, the bupivacaine can more easily elute from the device and cross the lipopholic cell membrane than can the ionic form of bupivacaine (for example, Bupivacaine.HCl). Once inside the cell, the non-ionic form will spontaneously convert to the ionic form because the local pH is below the pKa of bupivacaine. The pharmaceutically active form of bupivacaine, which acts by inhibiting sodium ion influx at the Node of Ranvier, is the ionic form.

Anesthetics having Ionic and Non-ionic Forms

The ionic form of such anesthetics typically contains a proton bound to a proton binding site, such as an ionic protonated quaternary amine structure. The ionic form exists in equilibrium with a non-ionic form in which the proton is absent from this site. In solution, the relationship between the percentage of the anesthetic existing in the ionic and non-ionic forms is given by the Henderson-Hasselbach equation,

$$pH = pKa + \log [\text{non-ionic form}]/ \text{ionic form}],$$

where [ionic form] refers to the concentration of the molecule in the protonated state, and [non-ionic form] refers to the concentration of the molecule in the deprotonated state.

Accordingly, when an anesthetic with an acidic proton binding site is placed in a solution at a pH = pKa of the acidic proton, 50% of the anesthetic exists in the protonated state and about 50% is in the deprotonated state. In one embodiment of the present invention, the "acidic proton binding site" can be any portion of a molecule (typically a nitrogen

atom) capable of covalently binding a proton ($H^+$) that is released at a pH of between about -1.76 and 15.76, preferably between about 0 and 14, and more preferably between 4 and 10.

Typically, the ionic form is more soluble in polar solvents such as water, while the non-ionic form can be more soluble in non-polar solvents. Frequently, anesthetics comprising a proton binding site are prescribed or provided in an ionic form. For example, bupivacaine is typically provided as the water-soluble formulation Bupivacaine·HCl, comprising bupivacaine in the ionic form (1) below.

Structure (2) below shows bupivacaine in the non-ionic form. This form is difficult to dissolve in water, but is more readily soluble in a variety of non-polar solvents. Upon conversion, the piperidine ring is protonated at the acidic proton binding site. The proton attached to the quaternary amine in bupivacaine structure (1) is an acidic proton with a pKa of about 8.09. Bupivacaine in non-ionic structure (2) is sparingly soluble in water. When bupivacaine is protonated, it forms ionic form (1), which is much more soluble in water.

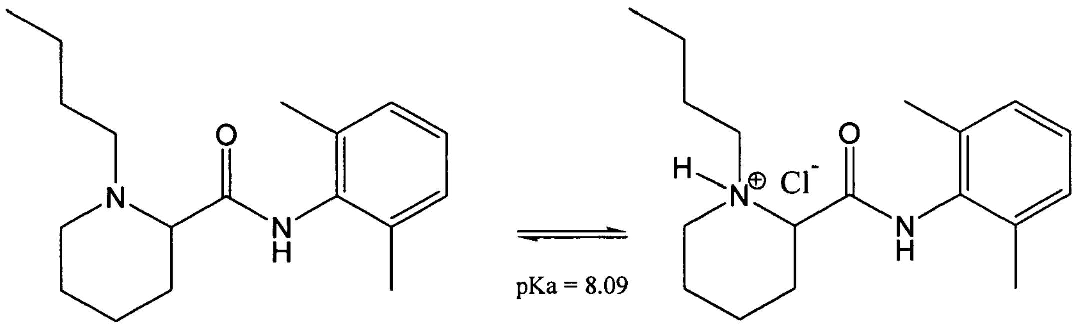


BP BP.HCl

1-Butyl-*N*-(2,6-dimethylphenyl)-2-piperidinecarboxamide

(Structure 2) (Structure 1)

There are other anesthetics having non-ionic forms that are relatively insoluble in water and ionic forms that are substantially more soluble in water. Such anesthetics include chloroprocaine, cocaine, lidocaine, mepivacaine, pramoxine and ropivacaine. Examples of ionic and non-ionic forms of some anesthetics are given in Table 1.

Table 1 - Anesthetics Having Protonated and Unprotonated Forms

| Name | Structure - unprotonated | Structure - protonated | Merck Index drug listing | pKa |
|---|---|---|---|---|
| bupivacaine | 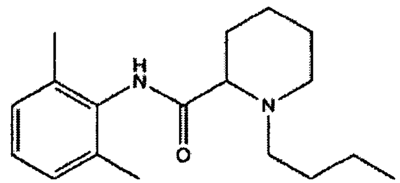 | 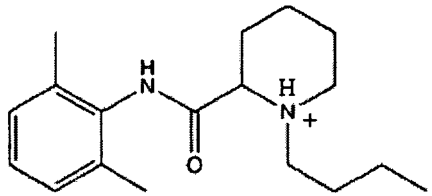 | Neutral HCl | 8.09 |
| lidocaine | 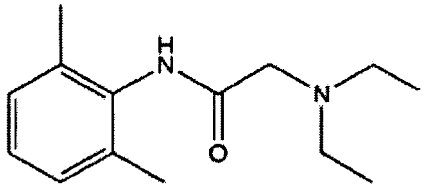 | 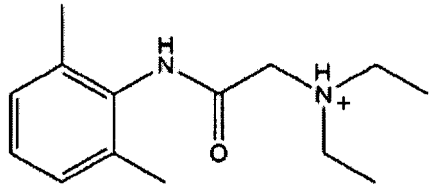 | Neutral HCl | 7.70 |
| mepivacaine | 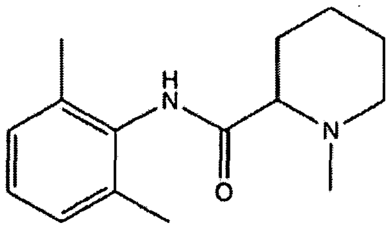 | 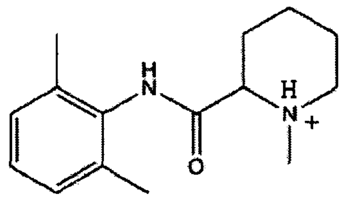 | Neutral HCl | 7.60 |
| ropivacaine | 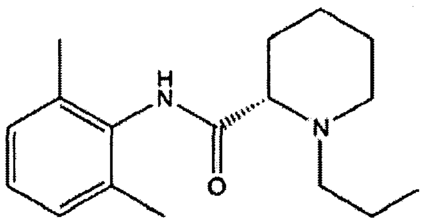 | 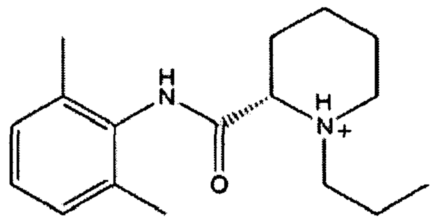 | Neutral HCl | 8.16 |

Preparation of the Non-ionic Form from the Ionic Form

Another aspect provides methods of incorporating an anesthetic into a medical device. The anesthetic preferably includes a proton binding site providing a pH dependent solubility in a solvent. The methods preferably include forming a solution comprising an anesthetic having a proton binding site for an acidic proton of a first pKa in water and contacting the solution with a medical device in a manner effective to incorporate the anesthetic into the medical device, for example by forming a coating containing the anesthetic on a surface of the medical device and/or by incorporating the anesthetic into a base material forming at least a portion of the medical device. The solution preferably has a pH that is controlled to provide a device comprising the anesthetic with the proton binding site in a form that is less soluble in water.

The anesthetic may be selected to have solubility in an aqueous solvent (e.g., water) that is comparatively higher when the proton binding site is in an ionic form compared to when the proton binding site is in a non-ionic form. Preferably, the proton binding site comprises nitrogen, such as a tertiary amine non-protonated non-ionic form and a quaternary amine cationic protonated form. In one embodiment, the anesthetic includes an amine proton binding site providing a pH dependent solubility in water. The solubility of the anesthetic in water may be higher when the proton binding site is in a protonated cationic form (e.g., a quaternary amine) than when the proton binding site is in a non-ionic form (e.g., a tertiary amine). Most preferably, the proton binding site is provided as a piperidine or other tertiary amine. The anesthetic may be a piperidine carboxanilide, including an anesthetic comprising a piperidine-2-carboxanilide of formula (1) above.

The solution may be obtained by dissolving the anesthetic with the proton binding site in a solvent at a desired pH. In one embodiment, the anesthetic may be provided in a multi-step process. Each proton binding site may bind an acidic proton characterized by a pKa in an aqueous solution depending on the pH of the solution. As described above, when the pH is equal to the pKa in solution, about 50% of the proton binding sites are in the protonated form. Raising the pH of a solution above the pKa increases the proportion of the anesthetic with the proton binding site in the unprotonated form compared to the protonated form according to the Henderson-Hasselbach equation above. Decreasing the pH of the first solution below the pKa decreases the proportion of the anesthetic with the proton binding site in the unprotonated form and increases the proportion of anesthetic molecules with protonated proton binding sites from the first solution. Additional increases in the pH of the solution will result in a greater proportion of the anesthetic being converted to the non-ionic form (e.g., precipitating out from the first solution).

For certain proton binding sites, such as nitrogen-containing binding sites, the protonated state is a cation and the unprotonated state is neutral or non-ionic. Therefore, increasing the pH of the coating solution (e.g., by adding a base) may decrease the solubility of the anesthetic in an aqueous solution, resulting in formation of a solid containing the anesthetic with the proton binding site in the unprotonated (non-ionic) form, which can be dissolved in a second solvent to form a coating solution. Therefore, the method may include (1) dissolving the anesthetic in a solvent (e.g., water) with the proton binding site in the ionic form to form a first solution; (2) adjusting the pH of the first solution to convert at least a fraction of proton binding sites to the non-ionic form, where the anesthetic is less soluble in the solvent when the proton binding site in the non-ionic form than in the ionic form; (3) isolating the fraction of the anesthetic with the proton binding site in the non-ionic form from the first solution; (4) dissolving the anesthetic with the proton binding site in the non-ionic form in a second solvent to form a coating solution and (5) coating the anesthetic in the coating solution onto or into a medical device to incorporate the anesthetic having the proton binding site in the non-ionic form.

For example, the bioactive bupivacaine.HCl having a quaternary (protonated) amine cationic proton binding site may be dissolved in water to form a clear first solution at a neutral pH (e.g., about pH 7). The pH of the solution may be lowered until bupivacaine with the proton binding site in the non-ionic deprotonated form precipitates as a white solid out of the first solution. The solid bupivacaine may be isolated from the first solution and dissolved in a suitable organic solvent, such as dichloromethane, and sprayed onto a medical device. The resulting device may have a reduced rate of elution of bupivacaine upon implantation within the urinary tract compared to a coating consisting of bupivacaine.HCl.

An aqueous solution of 4-8 g/L of the bupivacaine.HCl may be stirred and its pH raised from about neutral to a pH of about 9.5 to 10 (above the pKa of 8.09 of the acidic proton) by adding a small amount of a strong base, such as NaOH. As the base is added, the water-soluble protonated bupivacaine (formula (1)) is converted to the less soluble deprotonated form of bupivacaine (formula (2)), resulting in precipitation of the bupivacaine as a white solid. The precipitate may be washed and dissolved in an organic solvent as is discussed below.

Optionally, the anesthetics may be obtained directly with the proton binding site in a non-ionic form. Although many non-ionic anesthetics, such as bupivacaine, are minimally soluble in water, many may be dissolved in an organic solvent or in a non-polar solvent. Suitable solvents for use with bupivacaine include, but are not limited to, for methylene chloride (also known as dichloromethane, DCM), chloroform ($CHCl_3$), and tetrahydrofuran (THF). Other solvents may be used for the anesthetics useful in the present invention include, but are not limited to, alkanes such as pentane, hexane, octane, cyclohexane, heptane, isohexane, butane, pentane, isopentane, 2,2,4-trimethlypentane, nonane, decane, dodecane, hexadecane, eicosane, methylcyclohexane, cis-decahydronaphthalene, *N*-methyl pyrollidone and trans-decahydronaphthalene. Non-polar aromatic solvents may include benzene, toluene, xylene(s), naphthalene, styrene, ethylbenzene, 1-methylnaphthalene, 1,3,5-trimethylbenzene, tetrahydronaphthalene, diphenyl and 1,4-diethylbenzene, among others. Non-polar halohydrocarbon solvents may include chloro-hyhdrocarbons such as chloroform, methyl chloride, dichloromethane, 1,1-dichloroethylene, ethylene dichloride, ethylidene chloride, propyl chloride, cyclohexyl chloride, 1,1,1-trichloroethane, perchloroethylene, trichloroethylene, butyl chloride, carbon tetrachloride, tetrachloroethylene, chlorobenzene, o-dichlorobenzene, benzyl chloride, trichlorobiphenyl, methylcyclohexane, and 1,1,2,2-tetrachloroethane. Other non-polar solvents may include fluorinated halogenated species such as chlorodiflouoromethane, dichlorofluoromethane, dichlorodifluoromethane, trichlorofluoromethane, 1,2-dichlorotetrafluoroethane, 1,1,2-trichlorotrifluoroethane, perfluoro(methylcyclohexane), and perfluoro-(dimethylcyclohexane).

Other solvents include those with a polar portion, such as alcohols, so long as the solvent molecule itself is not polar, i.e., does not have a dipole moment or dielectric constant near that of water, or higher than that of water. Thus, water is clearly a polar solvent, as is dimethyl formamide (DMF), dimethyl sulfoxide (DMSO), hexamethylphosphoryl amide (HMPA), and hexamethyl phosphoryl triamide (HMPT). However, most organic solvents, such as ethyl acetate, diethyl ether, methyl alcohol, and n-butyl-alcohol, are considerably less polar than water. Organic solvents generally may be used.

In one embodiment, the non-ionic form of the anesthetic is relatively insoluble in water. Intrinsic water solubility for the non-ionic form of the anesthetic may be less than about 3%, 2% or 1 % by weight, and typically less than about 0.1% or 0.01% by weight. In one example, bupivacaine in its base or non-ionic form has a solubility of about 0.2 mg/mL, while the hydrochloride salt, bupivacaine hydrochloride, is described as being soluble in water.

Medical Devices Coated with the Non-ionic Form of an Anesthetic

Preferred medical devices of the present invention are manufactured from a non-metallic material. Such devices include polymeric or elastomeric urethral or ureteral stents or catheters. These devices are typically made from a silicone, polyurethane, or other polymeric material. Other suitable materials used in the medical device include carbon, carbon fiber, cellulose acetate, cellulose nitrate, polyethylene teraphthalate, silicone, polyurethane, polyamide, polyester, polyorthoester, polyanhydride, polyether sulfone, polycarbonate, polypropylene, high molecular weight polyethylene, polytetrafluoroethylene, a biocompatible polymeric material, polylactic acid, polyglycolic acid, polyanhydride, polycaprolactone, polyhydroxybutyrate valerate, a protein, an extracellular matrix component, collagen, fibrin or mixtures or copolymers thereof

In certain embodiments, the device is a stent, a graft, a stent graft, an implant, a guide wire, a balloon, a filter, a catheter, a Foley catheter or a cannula. In one preferred embodiment, the medical device is a ureteral stent.

**Figures 1-2** depict exemplary medical devices which may serve as substrates for coatings of the non-ionic form of the anesthetic. **Figure 1** depicts a Foley catheter, which may be implanted for weeks at a time. Such a device may incorporate an anesthetic and may also include another bioactive to resist infection or encrustation. Foley catheter **40** includes drainage shaft **41** with coating **42.** An expansion balloon **43** is placed in the bladder to retain the catheter. The Foley catheter may also include a drainage connector **44** on its proximal end **45,** and includes an inflation connector **46** for a balloon inflation lumen **47.** The expansion balloon **43** may also include a coating **42.** Coating **42** may include the non-ionic form of an anesthetic.

A pigtail-type ureteral stent is depicted in **Figures 2 and 2a.** Ureteral stent **50** includes distal and proximal ends **51 a** and intermediate length **53.** Orifices **52** allow urine to drain from the kidney into the stent. A portion of the stent may be coated with a coating **54** of an anesthetic.

Coating Methods

In one embodiment, an anesthetic is placed directly on the surface of the medical device and forms the outermost layer on the medical device. The anesthetic may be applied to the medical device in any known manner. For example, an anesthetic may be applied by spraying, dipping, pouring, pumping, brushing, wiping, vacuum deposition, vapor deposition, plasma deposition, electrostatic deposition, ultrasonic deposition, epitaxial growth, electrochemical deposition or any other method known to those skilled in the art.

In one embodiment, the layer of anesthetic contains from about 0.1 μg to about 100 μg of the anesthetic per $mm^2$ of the gross surface area of the structure. In another embodiment, the layer of anesthetic contains from about 1 μg to about 40 μg of the anesthetic per $mm^2$ of the gross surface area of the structure. "Gross surface area" refers to the area calculated from the gross or overall extent of the structure, and not necessarily to the actual surface area of the particular shape or individual parts of the structure. In other terms, about 100 μg to about 300 μg of anesthetic per 0.025 mm of coating thickness may be contained on the device surface.

In certain embodiments, the thickness of the coating layer is between 0.1 μm and 100 μm. In other embodiments, the thickness of the coating layer is between 0.1 μm and 10 μm. In yet other embodiments, the thickness of the coating layer is between 0.1 μm and 5 μm.

The incorporation of an anesthetic into the medical device will now be described using four illustrative methods: spray deposition, electrostatic deposition (ESD), ultrasonic deposition (USD) and immersion. However, it will be understood, that the anesthetic may be incorporated into the medical device using any known manner, including those mentioned above.

Spray Coating

In one embodiment, the anesthetic is dissolved in a suitable solvent(s) and sprayed onto the medical device under a fume hood using a spray gun, such as the Model Number 200 spray gun manufactured by Badger Air-Brush Company, Franklin Park, IL 60131. Alignment of the spray gun and medical device may be achieved with the use of laser beams, which may be used as a guide when passing the spray gun up and down the medical device.

Electrostatic Spray Deposition

In another embodiment, the anesthetic is dissolved in a suitable solvent and then sprayed onto the medical device using an electrostatic spray deposition (ESD) process. The ESD process generally depends on the principle that a charged particle is attracted towards a grounded target. Without being confined to any theory, the typical ESD process may be described as follows:

The solution that is to be deposited on the target is typically charged to several thousand volts (typically negative) and the target held at ground potential. The charge of the solution is generally great enough to cause the solution to jump across an air gap of several inches before landing on the target. As the solution is in transit towards the target, it fans out in a conical pattern which aids in a more uniform coating. In addition to the conical spray shape, the electrons are further attracted towards the conducting portions of the target, rather than towards the nonconductive base the target is mounted on, leaving the coating mainly on the target only.

In the ESD process, the anesthetic solution is forced through a capillary, which is subjected to an electrical field. The solvent mixture leaves the capillary in the form of a fine spray, the shape of which is determined by the electrical field. The medical device is then coated by placing it in the spray and allowing the solvent to evaporate, leaving the desired coating on the device.

The ESD method allows for control of the composition and surface morphology of the deposited coating. In particular, the morphology of the deposited coating may be controlled by appropriate selection of the ESD parameters, as set forth in WO 03/006180 (Electrostatic Spray Deposition (ESD) of biocompatible coatings on Metallic Substrates), the contents of which are incorporated by reference. For example, a coating having a uniform thickness and grain size, as well as a smooth surface, may be obtained by controlling deposition conditions such as deposition temperature, spraying rate, precursor solution, and bias voltage between the spray nozzle and the medical device being coated. The deposition of porous coatings is also possible with the ESD method.

Ultrasonic Spray Deposition

In another embodiment, the anesthetic is incorporated into the medical device using an ultrasonic spray deposition (USD) process. Ultrasonic nozzles employ high frequency sound waves generated by piezoelectric transducers which convert electrical energy into mechanical energy. The transducers receive a high frequency electrical input and convert this into vibratory motion at the same frequency. This motion is amplified to increase the vibration amplitude at an atomizing surface.

The ultrasonic nozzle is configured such that excitation of the piezoelectric crystals creates a longitudinal standing wave along the length of the nozzle. The ultrasonic energy originating from the transducers undergoes a step transition and amplification as the standing wave traverses the length of the nozzle. The nozzle is designed such that a nodal plane is located between the transducers. For ultrasonic energy to be effective for atomization, the nozzle tip must be located at an antinode, where the vibration amplitude is greatest. To accomplish this, the nozzle's length must be a multiple of a half-wavelength. In general, high frequency nozzles are smaller, create smaller drops, and consequently have smaller maximum flow capacity than nozzles that operate at lower frequencies.

Liquid introduced onto the atomizing surface absorbs some of the vibrational energy, setting up wave motion in the liquid on the surface. For the liquid to atomize, the vibrational amplitude of the atomizing surface must be carefully controlled. Below a critical amplitude, the energy is insufficient to produce atomized drops. If the amplitude is excessively high, cavitation occurs. Only within a narrow band of input power is the amplitude ideal for producing the nozzle's characteristic fine, low velocity mist. Since the atomization mechanism relies only on liquid being introduced onto the atomizing surface, the rate at which liquid is atomized depends solely on the rate at which it is delivered to the surface.

For example, the medical device, such as a stent, is coated using an ultrasonic spray nozzle, such as those available from Sono-Tek Corp., Milton, NY 12547. The solution is loaded into a 10.0 mL syringe, which is mounted onto

a syringe pump and connected to a tube that carries the solution to the ultrasonic nozzle. The syringe pump is then used to purge the air from the solution line and prime the line and spay nozzle with the solution. The stent is loaded onto a stainless steel mandrel in the ultrasonic coating chamber by the following method. The stent is held on a mandrel by silicone tubing at each end.

Immersion

In another embodiment, the medical device is immersed into an anesthetic solution until the proper dose is incorporated into the device. Methods for dip coating a medical device are disclosed in, for example, U.S. Patent Nos. 6,153,252 and 5,624,704.

In one example, ureteral stents are coated by sequentially dipping them into a solution of an anesthetic, drying, and then dipping again. The dipping process may be repeated as many times as desired until the desired coating depth is achieved. Alternatively, a stent may be dipped into a coating solution having a specific concentration of anesthetic for a sufficient time to incorporate the desired amount of anesthetic into the device. In certain embodiments, a lyophilizing process, with unitary or sequential cycles of applying the anesthetic and then freeze-drying and applying a vacuum, may also be used to remove the solvent and consolidate the coating.

A more complete understanding of the relative properties of an anesthetic (in this case bupivacaine) in the ionic and non-ionic forms can be obtained by reference to the following specific Examples. The Examples are described solely for purposes of illustration and are not intended to define the scope of the invention, which is set forth in the appended claims.

*Example 1: Preparation of Ureteral Stents containing Bupivacaine and Elution from the Stents in an Aqueous Medium*

An approximately 0.6% solution of bupivacaine having a tertiary amine in the non-ionic form (formula (2a) above) in dichloromethane was loaded into a syringe mounted onto a syringe pump and connected to a tube that carried the solution to an ultrasonic nozzle (Model 06-05108 - Sono-Tek Co., Poughkeepsie, NY 12547.) A nozzle was 6 mm diameter, 11 mm long and used with a nominal frequency of 120 kHz. Air was purged from the solution line and the spray nozzle primed with the solution. The coating chamber was purged with nitrogen to attain an oxygen concentration below 100 ppm. The solution was sprayed onto ureteral stent substrates 20 to 280 mm long (polyurethane double pigtail soflex ureteral stent, Cook Incorporated (Bloomington, IN)). A smooth, uniform coating was observed. Control stents were coated with the same concentration of Bupivacaine.HCl using the above coating method.

The stents were placed in solutions at different pH values to test the elution of bupivacaine over time. **Figure 3** shows the elution of bupivacaine at pH values of 4.5, 7.0 and 9.5 over a 12-day period. Acetic acid/sodium acetate was used for the pH 4.5 buffer, 0.1 M phosphate-buffered solution (PBS) for the pH 7.0 buffer, and sodium carbonate for the pH 9.5 buffer. In comparison, stents coated with Bupivacaine·HCl eluted fully within minutes in aqueous solutions.

**Figs. 4-7** depict photomicrographs of the coatings showing the uniformity and tenacity of the non-ionic coatings, in contrast to the water-soluble ionic coatings. **Figure 4** depicts a ureteral stent **80,** with left portion **81** coated with Bupivacaine·HCl; the portion on the right **82** was identically coated but was dipped in water for one minute and the coating is substantially dissolved. **Figure 5** depicts a relatively rough, crystalline coating **91** of Bupivacaine·HCl on a ureteral stent **90,** on the left, and the same stent after a one-minute ex *vivo* exposure to a pig ureter. Most of the coating **92** has been dissolved.

In contrast, **Figure 6** depicts another stent **100** coated with non-ionic bupivacaine **103** that has been exposed to an aqueous environment for several days, with virtually no effect on the coating, because as is well known, the non-ionic, neutral form of bupivacaine is only very slightly soluble in water. **Figure 7** then depicts the same type of stent **110** with a coating **114** of the non-ionic form of bupivacaine after 24 hours in an ex vivo test with a pig ureter. In contrast to the ionic form of the coating, most of the non-ionic form of the bupivacaine coating remains on the stent after 24 hours. This shows the durability of the coating and its resistance to dissolution or erosion by body fluids.

*Example 2: Immersion of Polymer Stent in a Solution of Bupivacaine and Methanol*

The pH of a typical coating solution used to coat a polyurethane drainage stent was measured. The pH of 1 L of methanol was measured at about 7.00. A polyurethane ureteral stent (SOF-FLEX, Cook Incorporated, Bloomington, IN) having a 6-French outer diameter, an inner diameter of 2 mm and a length of about 34 cm was placed in the methanol. After 1 hour, the pH was measured at about 6.35. A total of 235 g of bupivacaine base (formula (2a) above) was dissolved in the 1 L of methanol containing the ureteral stent. After 1 hour, the pH of the bupivacaine methanol solution in the presence of the ureteral stent was measured at about 10.00. Given the high pH, it is believed that most of the bupivacaine in the solution and in contact with the ureteral stent in the solution is in the non-protonated, non-ionic form that is less soluble in water.

*Example 3: Bupivacaine Coatings Applied to Polymer Stents by Immersion at High and Low Doses*

A series of eight polyurethane stents described in Example 2 were coated with bupivacaine by placing the stents in a coating solution containing methanol and bupivacaine. Four high dose coated stents were formed by placing the uncoated stents in a first solution containing 470 g bupivacaine base (100% saturation) in 1 liter of methanol for 100 minutes. Four low dose coated stents were formed by placing the uncoated stents in a second solution containing 235 g bupivacaine base in 1 liter of methanol (50% saturation) for 60 minutes. "Bupivacaine base" refers to bupivacaine obtained according to formula (2a) above (i.e., with the proton binding site in the unprotonated, non-ionic form).

The stents were removed, dried and the amount of bupivacaine retained on each stent was determined by immersing the stent in an aqueous dibasic buffer at a pH of 7.1 (0.1 M sodium phosphate) for a period of 24 hours, removing the stent and measuring the ultraviolet absorption of the aqueous dibasic buffer solution at 262 nm (using beer-lambert molar extinction coefficient of 427 l/mol cm, a 1cm cell length, a volume of 50 mL per sample), and correlating the UV absorption of the aqueous buffered solution to the amount of bupivacaine eluted from the stent over 24 hours in the sample. The stents were then transferred to another buffered solution and the UV measurement was repeated to determine the amount of bupivacaine eluting from the stent in the second 24 hour period. The "optical dose" of bupivacaine absorbed by each stent during immersion was calculated by UV detection and Beer-Lambert correlation of the absorbance due to bupivacaine that eluted from the stent into the buffered solution, and is shown in Table 2 for the low dose stents and Table 3 for the high dose stents. The "gravimetric dose" was determined by weighing the stents before immersion and after drying.

**Table 2. Mass Balances. 50% Saturation, 60 min**

| Stent 50/60 Mass Balance | | | |
|---|---|---|---|
| Article | Grav. Dose BP (mg) | Optical Dose BP (mg) | Mass Balance |
| 5 | 64.84 | 68.61 | 105.81% |
| 10 | 64.61 | 68.94 | 106.70% |
| 19 | 62.40 | 67.91 | 108.83% |
| 20 | 63.58 | 68.84 | 108.27% |

**Table 3. Mass Balances, 100% Saturation, 100 min**

| Stent 100/100 Mass Balance | | | |
|---|---|---|---|
| article | Grav. Dose BP (mg) | Optical Dose BP (mg) | Mass Balance |
| 25 | 160.48 | 166.96 | 104.04% |
| 27 | 162.32 | 172.41 | 106.22% |
| 37 | 154.47 | 161.92 | 104.82% |
| 39 | 157.87 | 165.68 | 104.95% |

The amount of bupivacaine that eluted from each of the eight stents over a period of 28 days (low dose) and 38 days (high dose) into a buffered aqueous solution at a pH of about 7 is shown in **FIG. 8.** The four high dose samples provided a first group of elution curves 100 showing elution of about 180 mg of bupivacaine within about 38 days; the four low dose samples provided a second group of elution curves 200 showing elution of up to about 60 mg of bupivacaine in about 28 days.

## Claims

**1.** A non-metallic medical device comprising:

a non-metallic substrate; and
an anesthetic in contact with the non-metallic substrate, the anesthetic having a proton binding site with a non-ionic form and an ionic form, the anesthetic being less soluble in water when the proton binding site is in the

non-ionic form than when the proton binding site is in the ionic form, wherein at least 5% w/w of the anesthetic is present with the proton binding site in the non-ionic form and the remainder of the anesthetic is present with the proton binding site in the ionic form.

2. The non-metallic medical device of claim 1, wherein at least 95% w/w of the anesthetic is present with the proton binding site in the non-ionic form and the remainder of the anesthetic is present with the proton binding site in the ionic form.

3. The non-metallic medical device of claim 1, wherein the proton binding site has a protonated ionic form and an unprotonated non-ionic form.

4. The non-metallic medical device of claim 3, wherein the proton binding site includes a nitrogen atom.

5. The medical device of claim 4, wherein the proton binding site is a tertiary amine in the non-ionic form and a cationic quaternary amine in the ionic form.

6. The medical device of claim 1, wherein the anesthetic comprises a chemical structure of the formula:

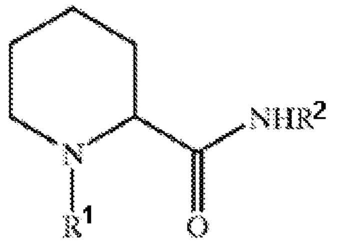

where $R^1$ is alkyl and $R^2$ is an optionally alkyl substituted phenyl.

7. The non-metallic medical device of claim 1, wherein the anesthetic is selected from the group consisting of bupivacaine, chloroprocaine, cocaine, lidocaine, mepivacaine, pramoxine and ropivacaine.

8. The non-metallic medical device of claim 1, wherein the non-metallic substrate comprises a material selected from the group consisting of carbon, carbon fiber, cellulose acetate, cellulose nitrate, polyethylene teraphthalate, silicone, polyurethane, polyamide, polyester, polyorthoester, polyanhydride, polyether sulfone, polycarbonate, polypropylene, high molecular weight polyethylene, polytetrafluoroethylene, a biocompatible polymeric material, polylactic acid, polyglycolic acid, polyanhydride, polycaprolactone, polyhydroxybutyrate valerate, a protein, an extracellular matrix component, collagen, fibrin and mixtures and copolymers thereof.

9. The non-metallic medical device of claim 1 comprising a ureteral stent.

10. A method for producing the medical device of Claim 1, the method comprising:

(1) dissolving the anesthetic in a solvent with the proton binding site in the ionic form to form a first solution;
(2) adjusting the pH of the first solution to convert at least a fraction of proton binding sites to the non-ionic form, where the anesthetic is less soluble in the solvent when the proton binding site in the non-ionic form than in the ionic form;
(3) isolating the fraction of the anesthetic with the proton binding site in the non-ionic form from the first solution;
(4) dissolving the anesthetic with the proton binding site in the non-ionic form in a second solvent to form a coating solution; and
(5) coating the anesthetic in the coating solution onto or into the medical device to incorporate the anesthetic having the proton binding site in the non-ionic form.

11. The method of claim 10, wherein the ionic form of the proton binding site is protonated and the non-ionic form of the proton binding site is deprotonated.

12. The method of claim 10, wherein the proton binding site includes a nitrogen atom.

**13.** The method of claim 10, wherein the anesthetic comprises a chemical structure of the formula:

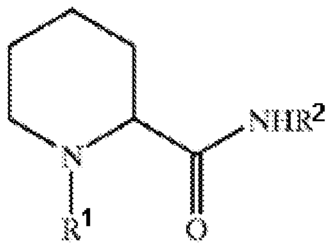

where the N bonded to $R^1$ is the proton binding site, $R^1$ is alkyl and $R^2$ is an optionally alkyl substituted phenyl.

**14.** The method of claim 10, wherein non-metallic substrate comprises a polyurethane and the anesthetic is preferably bupivacaine.

## Patentansprüche

**1.** Nichtmetallische medizinische Vorrichtung, umfassend:

ein nichtmetallisches Substrat und
ein Anästhetikum in Kontakt mit dem nichtmetallischen Substrat, wobei das Anästhetikum eine Protonenbindungsstelle mit einer nichtionischen Form und einer ionischen Form aufweist, wobei das Anästhetikum weniger löslich in Wasser ist, wenn die Protonenbindungsstelle in der nichtionischen Form vorliegt, als wenn die Protonenbindungsstelle in der ionischen Form vorliegt, wobei mindestens 5 % Gew./Gew. des Anästhetikums mit der Protonenbindungsstelle in der nichtionischen Form vorliegen und der Rest des Anästhetikums mit der Protonenbindungsstelle in der ionischen Form vorliegt.

**2.** Nichtmetallische medizinische Vorrichtung nach Anspruch 1, wobei mindestens 95 % Gew./Gew. des Anästhetikums mit der Protonenbindungsstelle in der nichtionischen Form vorliegen und der Rest des Anästhetikums mit der Protonenbindungsstelle in der ionischen Form vorliegt.

**3.** Nichtmetallische medizinische Vorrichtung nach Anspruch 1, wobei die Protonenbindungsstelle eine protonierte ionische Form und eine unprotonierte nichtionische Form aufweist.

**4.** Nichtmetallische medizinische Vorrichtung nach Anspruch 3, wobei die Protonenbindungsstelle ein Stickstoffatom einschließt.

**5.** Medizinische Vorrichtung nach Anspruch 4, wobei die Protonenbindungsstelle ein tertiäres Amin in der nichtionischen Form und ein kationisches quaternäres Amin in der ionischen Form ist.

**6.** Medizinische Vorrichtung nach Anspruch 1, wobei das Anästhetikum eine chemische Struktur mit der Formel:

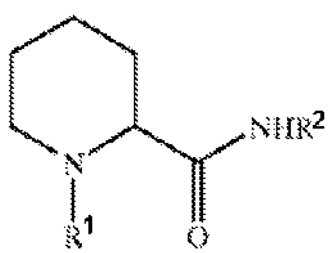

umfasst, wobei $R^1$ Alkyl ist und $R^2$ ein gegebenenfalls alkylsubstituiertes Phenyl ist.

**7.** Nichtmetallische medizinische Vorrichtung nach Anspruch 1, wobei das Anästhetikum ausgewählt ist aus der Gruppe bestehend aus Bupivacain, Chlorprocain, Kokain, Lidocain, Mepivacain, Pramoxin und Ropivacain.

8. Nichtmetallische medizinische Vorrichtung nach Anspruch 1, wobei das nichtmetallische Substrat ein Material ausgewählt aus der Gruppe bestehend aus Kohle, Kohlefaser, Celluloseacetat, Cellulosenitrat, Polyethylenterephthalat, Silikon, Polyurethan, Polyamid, Polyester, Polyorthoester, Polyanhydrid, Polyethersulfon, Polycarbonat, Polypropylen, Polyethylen mit hohem Molekulargewicht, Polytetrafluorethylen, einem biokompatiblen polymeren Material, Polymilchsäure, Polyglykolsäure, Polyanhydrid, Polycaprolacton, Polyhydroxybutyratvalerat, einem Protein, einer extrazellulären Matrixkomponente, Kollagen, Fibrin und Mischungen und Copolymeren davon umfasst.

9. Nichtmetallische medizinische Vorrichtung nach Anspruch 1, umfassend eine Harnleiterschiene.

10. Verfahren zur Herstellung der medizinischen Vorrichtung nach Anspruch 1, wobei das Verfahren umfasst:

(1) Lösen des Anästhetikums mit der Protonenbindungsstelle in der ionischen Form in einem Lösungsmittel, um eine erste Lösung zu bilden;
(2) Einstellen des pH-Werts der ersten Lösung, um mindestens einen Teil der Protonenbindungsstellen in die nichtionische Form umzuwandeln, wobei das Anästhetikum in dem Lösungsmittel weniger löslich ist, wenn die Protonenbindungsstelle in der nichtionischen Form vorliegt, verglichen mit der ionischen Form;
(3) Isolieren des Teils des Anästhetikums mit der Protonenbindungsstelle in der nichtionischen Form aus der ersten Lösung;
(4) Lösen des Anästhetikums mit der Protonenbindungsstelle in der nichtionischen Form in einem zweiten Lösungsmittel, um eine Beschichtungslösung zu bilden, und
(5) Aufbringen des Anästhetikums in der Beschichtungslösung als Beschichtung auf oder in die medizinische Vorrichtung, um das Anästhetikum mit der Protonenbindungsstelle in der nichtionischen Form einzubauen.

11. Verfahren nach Anspruch 10, wobei die ionische Form der Protonenbindungsstelle protoniert ist und die nichtionische Form der Protonenbindungsstelle deprotoniert ist.

12. Verfahren nach Anspruch 10, wobei die Protonenbindungsstelle ein Stickstoffatom einschließt.

13. Verfahren nach Anspruch 10, wobei das Anästhetikum eine chemische Struktur mit der Formel:

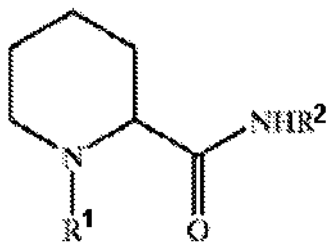

umfasst, wobei das an $R^1$ gebundene N die Protonenbindungsstelle ist, $R^1$ Alkyl ist und $R^2$ ein gegebenenfalls alkylsubstituiertes Phenyl ist.

14. Verfahren nach Anspruch 10, wobei das nichtmetallische Substrat ein Polyurethan umfasst und das Anästhetikum vorzugsweise Bupivacain ist.

**Revendications**

1. Dispositif médical non métallique comprenant :

un substrat non métallique ; et
un anesthésique en contact avec le substrat non métallique, l'anesthésique ayant un site de liaison protonique avec une forme non ionique et une forme ionique, l'anesthésique étant moins soluble dans l'eau lorsque le site de liaison protonique est sous la forme non ionique que lorsque le site de liaison protonique est sous la forme ionique, au moins 5 % pds/pds de l'anesthésique étant présent avec le site de liaison protonique sous la forme non ionique et le reste de l'anesthésique étant présent avec le site de liaison protonique sous la forme ionique.

2. Dispositif médical non métallique de la revendication 1, dans lequel au moins 95 % pds/pds de l'anesthésique est

présent avec le site de liaison protonique sous la forme non ionique et le reste de l'anesthésique est présent avec le site de liaison protonique sous la forme ionique.

3. Dispositif médical non métallique de la revendication 1, dans lequel le site de liaison protonique a une forme ionique protonée et une forme non ionique non protonée.

4. Dispositif médical non métallique de la revendication 3, dans lequel le site de liaison protonique comporte un atome d'azote.

5. Dispositif médical de la revendication 4, dans lequel le site de liaison protonique est une amine tertiaire sous la forme non ionique et une amine quaternaire cationique sous la forme ionique.

6. Dispositif médical de la revendication 1, dans lequel l'anesthésique comprend une structure chimique de la formule :

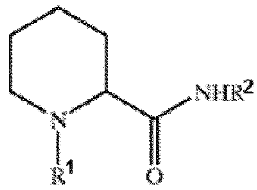

où $R^1$ est un alkyle et $R^2$ est un phényle éventuellement substitué par un alkyle.

7. Dispositif médical non métallique de la revendication 1, dans lequel l'anesthésique est choisi dans le groupe constitué par la bupivacaïne, la chloroprocaïne, la cocaïne, la lidocaïne, la mépivacaïne, la pramoxine et la ropivacaïne.

8. Dispositif médical non métallique de la revendication 1, dans lequel le substrat non métallique comprend un matériau choisi dans le groupe constitué par le carbone, la fibre de carbone, l'acétate de cellulose, le nitrate de cellulose, le téréphtalate de polyéthylène, le silicone, le polyuréthane, le polyamide, le polyester, le polyorthoester, le polyanhydride, la polyéthersulfone, le polycarbonate, le polypropylène, le polyéthylène à haut poids moléculaire, le polytétrafluoroéthylène, un matériau polymère biocompatible, l'acide polylactique, l'acide polyglycolique, le polyanhydride, la polycaprolactone, le polyhydroxybutyrate-valérate, une protéine, un constituant de la matrice extracellulaire, le collagène, la fibrine et les mélanges et copolymères de ceux-ci.

9. Dispositif médical non métallique de la revendication 1 comprenant un stent urétéral.

10. Procédé de production du dispositif médical de la revendication 1, le procédé comprenant les étapes suivantes :

(1) dissoudre l'anesthésique dans un solvant avec le site de liaison protonique sous la forme ionique pour former une première solution ;
(2) ajuster le pH de la première solution pour transformer au moins une fraction des sites de liaison protonique dans la forme non ionique, l'anesthésique étant moins soluble dans le solvant quand le site de liaison protonique est sous la forme non ionique que sous la forme ionique ;
(3) isoler la fraction de l'anesthésique avec le site de liaison protonique sous la forme non ionique à partir de la première solution ;
(4) dissoudre l'anesthésique avec le site de liaison protonique sous la forme non ionique dans un deuxième solvant pour former une solution de revêtement ; et
(5) déposer l'anesthésique dans la solution de revêtement sur ou dans le dispositif médical pour incorporer l'anesthésique ayant le site de liaison protonique sous la forme non ionique.

11. Procédé de la revendication 10, dans lequel la forme ionique du site de liaison protonique est protonée et la forme non ionique du site de liaison protonique est déprotonée.

12. Procédé de la revendication 10, dans lequel le site de liaison protonique comporte un atome d'azote.

13. Procédé de la revendication 10, dans lequel l'anesthésique comprend une structure chimique de la formulé :

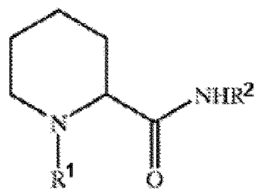

où le N lié à $R^1$ est le site de liaison protonique, $R^1$ est un alkyle et $R^2$ est un phényle éventuellement substitué par un alkyle.

**14.** Procédé de la revendication 10, dans lequel le substrat non métallique comprend un polyuréthane et l'anesthésique est de préférence la bupivacaïne.

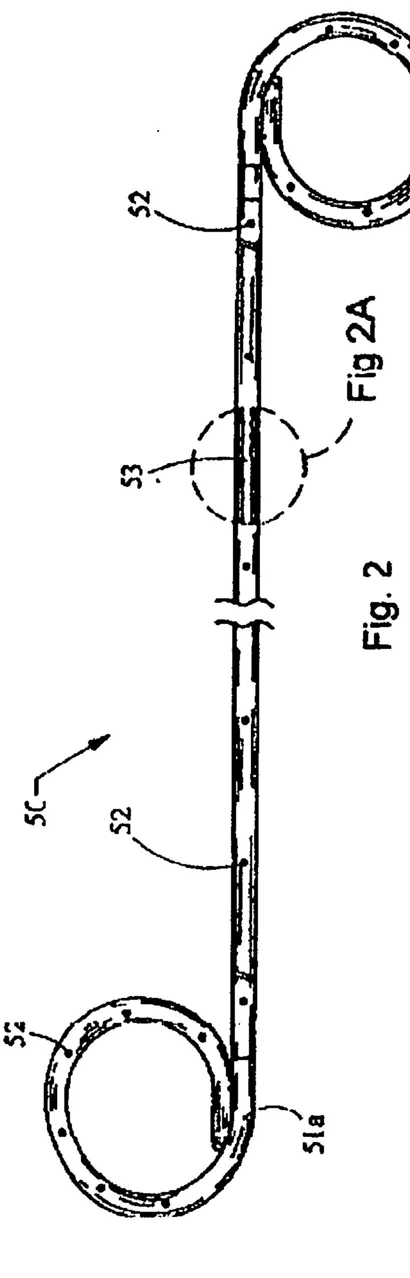


Fig. 2

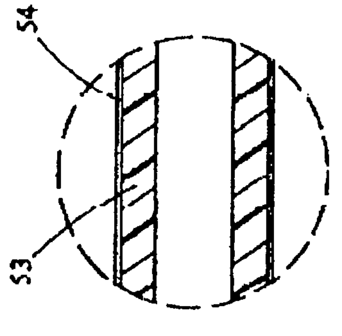


Fig. 2A

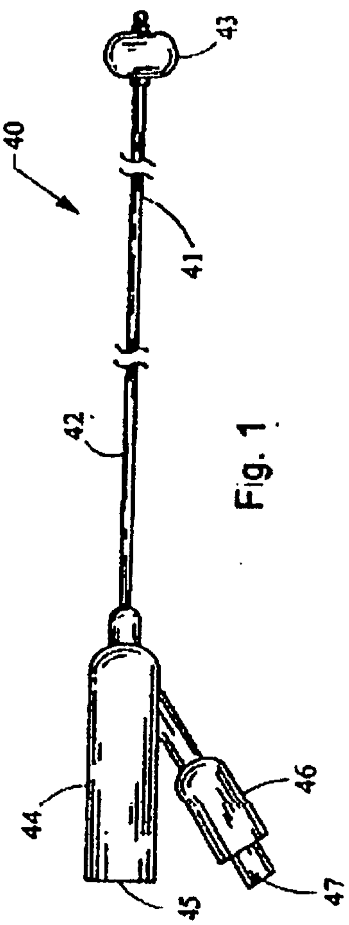


Fig. 1

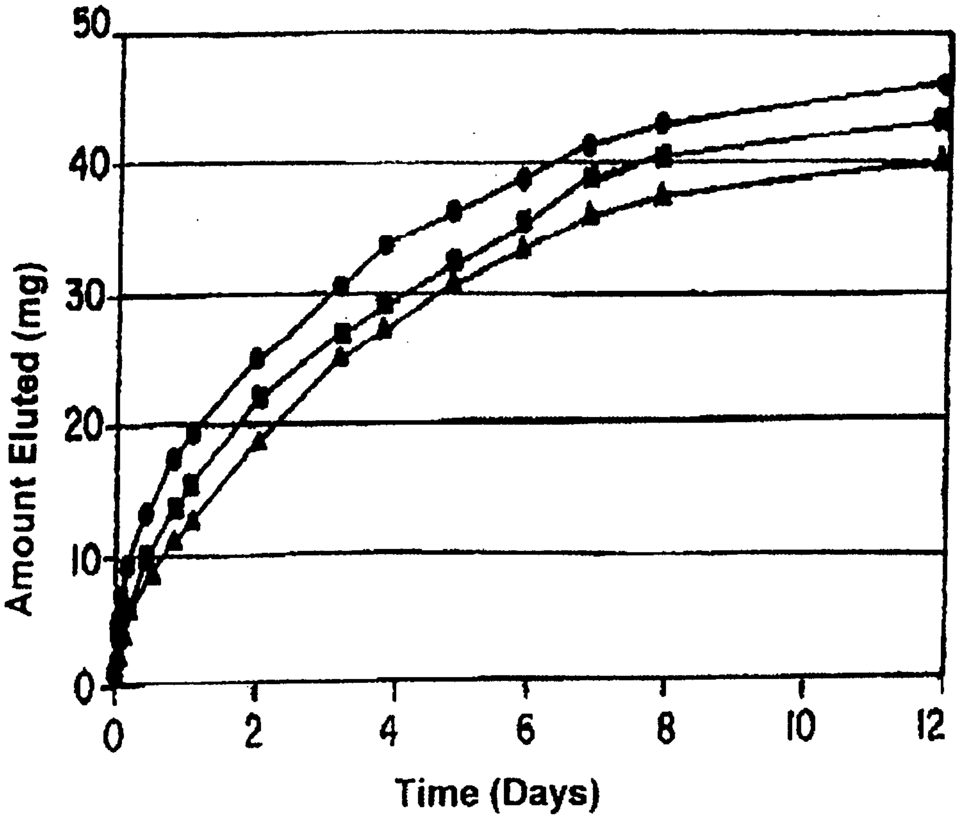


Fig. 3

Fig. 4

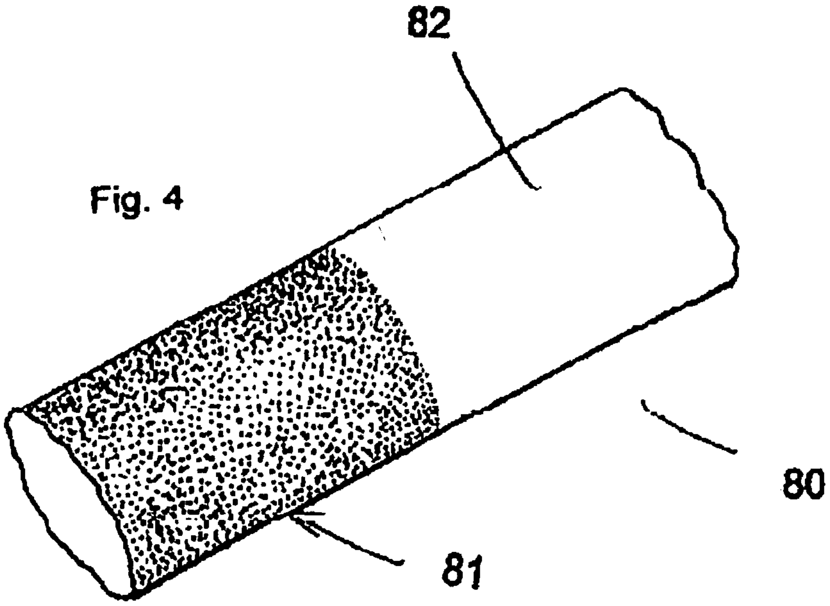


Prior Art

Prior Art

Fig. 5

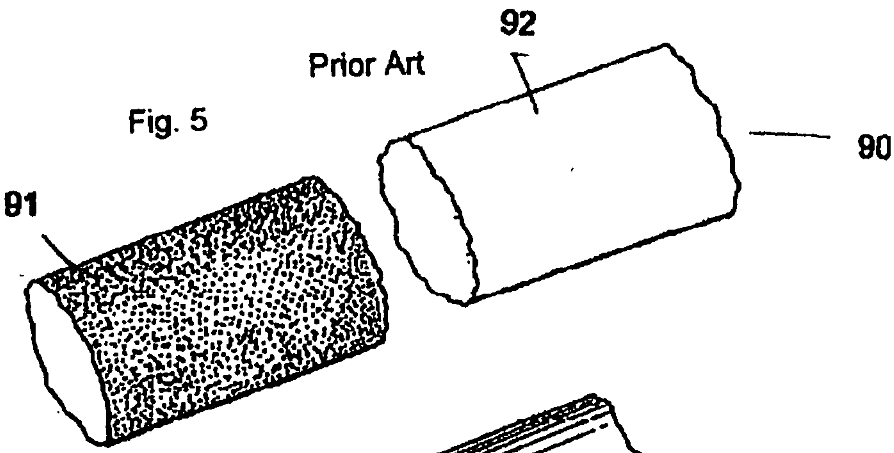


Fig. 6

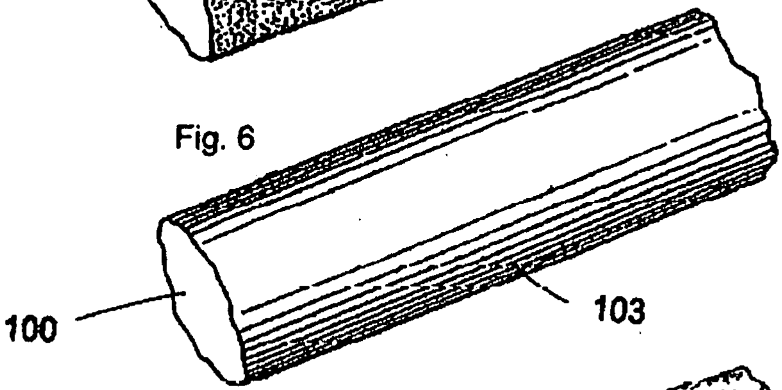


Fig.7

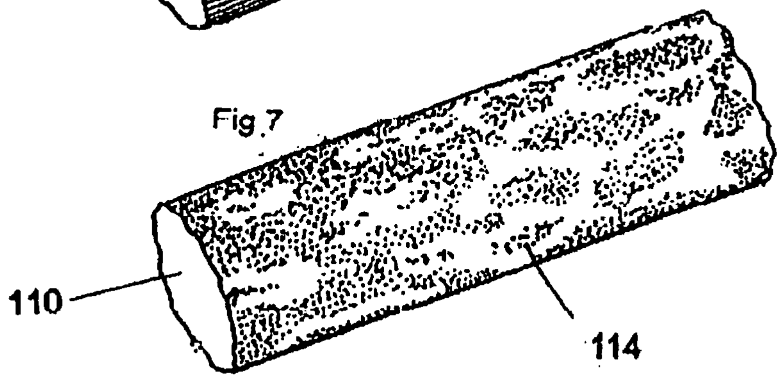

Fig. 8

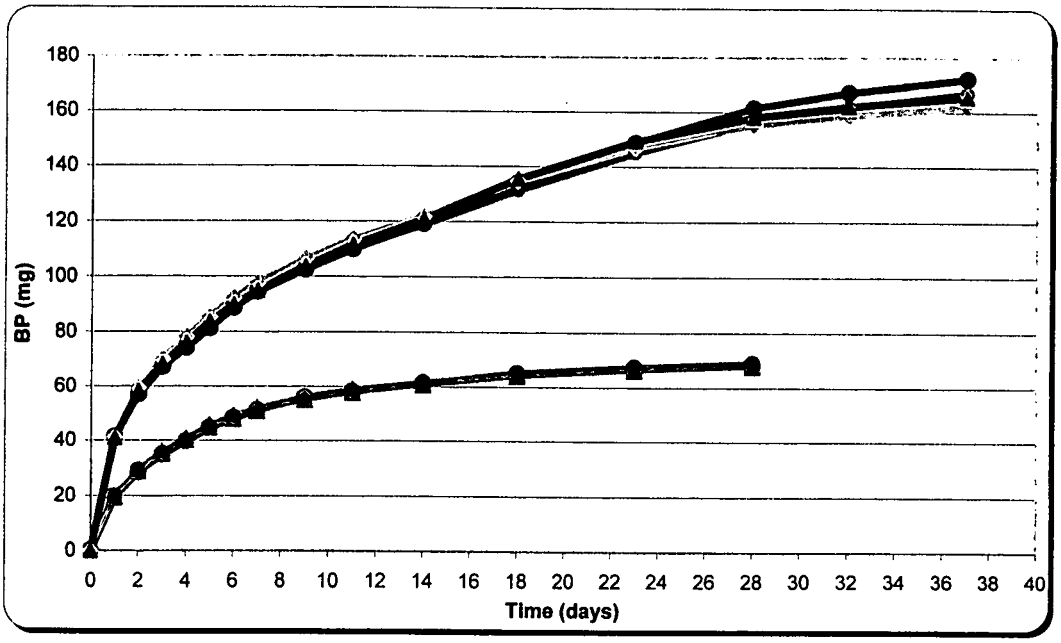
180
160
140
120
100
80
60
40
20
0
BP (mg)
0
2
4
6
8
10
12
14
16
18
20
22
24
26
28
30
32
34
36
38
40
Time (days)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4795644 A
- US 5759708 A
- US 5958430 A
- US 20050025791 A1
- WO 03006180 A
- US 6153252 A
- US 5624704 A